Europäisches Patentamt

European Patent Office

Office européen des brevets

(11) Publication number: **0 094 964**
**B1**

# EUROPEAN PATENT SPECIFICATION

(45) Date of publication of patent specification: **10.05.89**

(51) Int. Cl.⁴: **A 61 B 1/06, B 23 K 9/00**

(21) Application number: **83900156.7**

(22) Date of filing: **30.11.82**

(86) International application number:
**PCT/US82/01669**

(87) International publication number:
**WO 83/01893 09.06.83 Gazette 83/14**

(54) **A CATHETER ASSEMBLY.**

(30) Priority: **01.12.81 US 326221**

(43) Date of publication of application:
**30.11.83 Bulletin 83/48**

(45) Publication of the grant of the patent:
**10.05.89 Bulletin 89/19**

(84) Designated Contracting States:
**DE FR GB**

(56) References cited:
**FR-A-2 278 305**
**US-A-3 470 876**
**US-A-3 858 577**
**US-A-3 866 599**
**US-A-3 900 022**
**US-A-4 146 019**
**US-A-4 175 545**
**US-A-4 207 874**
**US-A-4 222 375**
**US-A-4 224 929**
**US-A-4 269 485**
**US-A-4 290 421**
**US-A-4 295 464**

(73) Proprietor: **THE REGENTS OF THE UNIVERSITY OF CALIFORNIA**
**2490 Channing Way**
**Berkeley California 94720 (US)**

(72) Inventor: **GARRETT, Lee Cedars Medical Center**
**Cedars South, Suite K 1295 NW 14th Street**
**Miami, FL 33125 (US)**

(74) Representative: **Bayliss, Geoffrey Cyril et al**
**BOULT, WADE & TENNANT 27 Furnival Street**
**London EC4A 1PQ (GB)**

(56) References cited:
**US-A-4 299 226**
**US-A-4 313 431**
**US-A-4 327 720**
**US-A-4 418 688**

## Description

The present invention relates generally to catheter assemblies, and more particularly to a catheter assembly for diagnostic use or for use in removing an obstruction in a blood vessel, body channel or body cavity.

The American Heart Association has estimated that approximately four million people in the United States suffer from arteriosclerotic coronary artery disease. Many of these people are likely to suffer or die from a myocardial infraction, commonly known as a heart attack. Heart attacks are in fact the leading cause of death in the United States. Thrombosis in the coronary artery beyond the arteriosclerotic constriction is the usual cause of heart attacks. A procedure which can open arteriosclerotic constrictions, thereby permitting the normal flow of blood to the heart, may reduce the many deaths and disabilities caused by heart disease.

Constrictions in the coronary artery are caused by a build-up of plaque, which may be "hard" or "soft". Plaque consists of calcium, fibrous and fatty substances. If the plaque is of recent origin or "soft", that is, it has a low concentration of calcium, a "Grüntzig" balloon catheter may be used to clear the artery. The "Grüntzig" catheter is inserted into the constricted area of the artery, and the balloon is inflated to expand and compress the plaque, thereby opening the artery and permitting an increased flow of blood through the artery. The "Grüntzig" balloon catheter is described in the following article: "Nonoperative Dilatation of Coronary-Artery Stenosis", A. R. Grüntzig, M.D., A. Senning, M.D., and W. E. Siegenthaler, M.D., The New England Journal of Medicine, Vol. 301, No. 2, July 12, 1979. See also U.S. Patent No. 4,195,637, Grüntzig, et al., issued April 1, 1980.

The "Grüntzig" technique, however, does not work where the constriction in the artery is very tight, where the plaque is "old" and hard, or where the plaque forming the constriction has a high concentration of calcium and thus is very hard. It is estimated that the "Grüntzig" technique can be successfully used on only about 5% of the patients suffering from arteriosclerotic coronary disease.

Accordingly, the present invention is directed to a catheter assembly which can remove (a) plaque, especially when hard, and (b) operate in a very constricted area of an artery. The catheter assembly of the present invention may also be used to remove obstructions from other blood vessels and body channels or cavities, as well as to view a region of a blood vessel or a non-vascular body channel.

U.S.-A-3858577 and U.S.-A-4146019 disclose optical fibers for illimination, viewing and laser transmission. Laser energy is utilised to coagulate blood or cauterize a wound, typically in the stomach. Neither the '577 or the '019 Patent discloses the use of a catheter assembly which is able to interrupt temporarily the flow of blood to an occluded or constricted region whilst such region is treated or observed.

This invention provides a catheter assembly, comprising an outer catheter for insertion into a body channel, said outer catheter having distal and proximal ends, an inner catheter positionable within said outer catheter and having a distal end that is extendable toward the distal end of said outer catheter, an optical fiber extending through a portion or said inner catheter for transmitting laser energy therethrough for removing an obstruction outwardly of the distal end of said inner catheter, and optical fibers extending through a portion of said inner catheter for illuminating and viewing the area in front of the distal end of said inner catheter, wherein an inflatable means associated with one of said catheters is provided for effecting a seal with the interior wall of the body channel to prevent the flow of a body fluid therepast so as to facilitate laser irradiation of the obstruction outwardly of the distal end of said inner catheter.

Preferably means are provided for positioning said optical fibers relative to the axis of the inner catheter. For said positioning means may include a cable and pulley arrangement.

In any of the above arrangements said optical fibers for illuminating and viewing may include a plurality of optical fibers extending through a portion of said inner catheter and terminating within said inner catheter near the distal end thereof for use in illuminating the area in front of the distal end of said inner catheter; and a fiber optic bundle extending through a portion of said inner catheter and terminating within said inner catheter near the distal end thereof for use in viewing the area in front of the distal end of said inner catheter.

By way of example said optical fibers comprise a fiber optical bundle having a viewing bundle portion for viewing the area in front of the distal end of said inner catheter, and a light source bundle portion for illuminating said area.

More particularly there may be two of said fiber optical bundles disposed on opposite sides of the central axis of said inner catheter.

In a further arrangement said laser fiber may be positioned along the central axis of said inner catheter.

The catheter assembly of any of the above arrangements may include a flushing channel extending through a portion of said inner catheter and terminating within said inner catheter near the distal end thereof.

The catheter assembly may also include a suction channel extending through a portion of said inner catheter and terminating within said inner catheter near the distal end thereof.

In the latter arrangement the end of said laser fiber terminating near the distal end of said inner catheter may include a lens for focusing the laser beam on the obstruction.

The assembly may further include means for positioning said laser optical fiber relative to the central axis of said inner catheter. For example said positioning means for said laser optical fiber may include a cable and pulley arrangement.

Thus catheter assembly embodying the present invention may include fiber optics for viewing and illuminating a body region, a fiber optic for transmitting a laser beam to a region within the patient's body, or both types of fiber optics, as desired. Alternatively, both types of fiber optices may be omitted and the catheter assembly of the present invention used to pretreat the plaque, occlusion or clot with a chemotherapeutic substance. In this latter embodiment, the catheter assembly could be guided to the occlusion or clot by such means as fluoroscopy. Flouroscopy could also be used to guide the catheter assembly to the target area where the assembly includes only a laser fiber optic.

The catheter assembly of the present invention will be described in more detail hereinafter in conjunction with the drawings wherein:

Figures 1A through 1C are schematic, side views partly in section, illustrating the positioning of the inner catheter within the outer catheter of the catheter assembly of the present invention.

Figure 2 is a schematic, sectional view of the catheter assembly of the present invention.

Figure 3 is a schematic, sectional view of the catheter assembly of the present invention.

Figure 4 is a schematic, sectional view of an alternative embodiment of the catheter assembly of the present invention.

Figure 5 and 6 are schematic views, partly in section, of a positioning means used with the catheter assembly of the present invention.

Figure 7 is a schematic, sectional view along the central axis of the inner catheter illustrating an alternative embodiment of the catheter assembly of the present invention.

Figure 8 is a schematic, side view of the catheter assembly of the present invention wherein the inflatable means is affixed to the outer surface of the outer catheter.

The present invention will be described in conjunction with its most suitable use: the removal of plaque material from the coronary artery. The catheter assembly of the present invention, however, can also be used to remove occlusions, such as clots, in other arteries and veins. The present invention is especially useful in very constricted areas of blood vessels and for removing very hard material. Further, the catheter assembly could be used where it is necessary to temporarily stop the flow of blood to permit visual inspection and to achieve successful removal of an occlusion. The catheter assembly could also be used without its associated laser apparatus where it is simply desired to view an area of a blood vessel, some non-vascular body channel, or the interior of an organ or cavity. The present invention could also be used to remove obstructions from non-vascular body channels; for example, it could be used to remove bladder, kidney and gall stones.

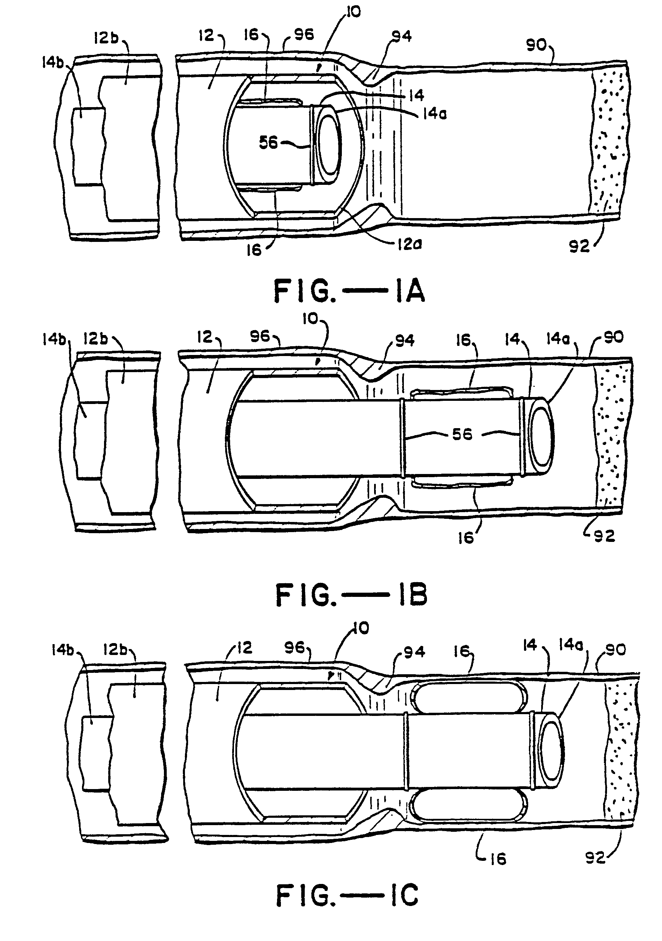

Referring now to the drawings, in which like components are designated by like reference numerals throughout the various figures, attention is first directed to Figures 1A through 1C. Figures 1A through 1C show catheter assembly 10 of the present invention positioned for use in removing plaque 92 from coronary artery 90. Catheter assembly 10 comprises a guide or outer catheter 12 and a second or inner catheter 14. Guide catheter 12 has been inserted into an arm, leg or other artery 96 to extend near orifice 94 of the coronary artery. The guide catheter is guided through such artery to the orifice by procedures well known in the art, such as fluoroscopy. Alternatively, catheter 12 may be an existing preformed catheter or articulated endoscope, adapted as described herein.

Catheter 14 is positioned in coronary artery 90 by inserting it through guide catheter 12. Catheter 12 guides catheter 14 from the point of insertion, which is at the proximal end 12b of catheter 12, into coronary artery 90. To facilitate movement of catheter 14 within guide catheter 12, catheter 14 is more flexible than catheter 12. Catheter 14 also has a smaller outside diameter than the guide catheter. The outside diameter of catheter 12 is about 2.5 to 3.5 millimeters for use within the coronary artery. For other blood vessels or body channels, the outside diameter of catheter 12 may be selected accordingly. The outside diameter of catheter 14 is approximately 1.5 to 2.5 millimeters. In instances, where the catheter assembly does not include optical fibers for viewing and illuminating, the outside diameter of catheter 14 can be as small as about 1 to 2 millimeters.

In use, catheter 14 is inserted in and pushed through the guide catheter until its distal end 14a extends beyond distal end 12a of the guide catheter, see Figures 1A through 1C. Inflatable means 16, discussed in more detail below, is affixed to inner catheter 14 at the distal end thereof. The inflatable means is collapsed as the inner catheter moves through the guide catheter, see Figures 1A and 1B. After distal end 14a of the inner catheter has been positioned near plaque buildup 92 by such means as fluoroscopy and the viewing and illuminating fiber optics, which are discussed below, inflatable means 16 is inflated as shown in Figure 1C. Radio-opaque bands 56 may be located at distal end 14a so that the position of distal end 14a and inflatable means 16 can be precisely determined by use of the fluoroscope.

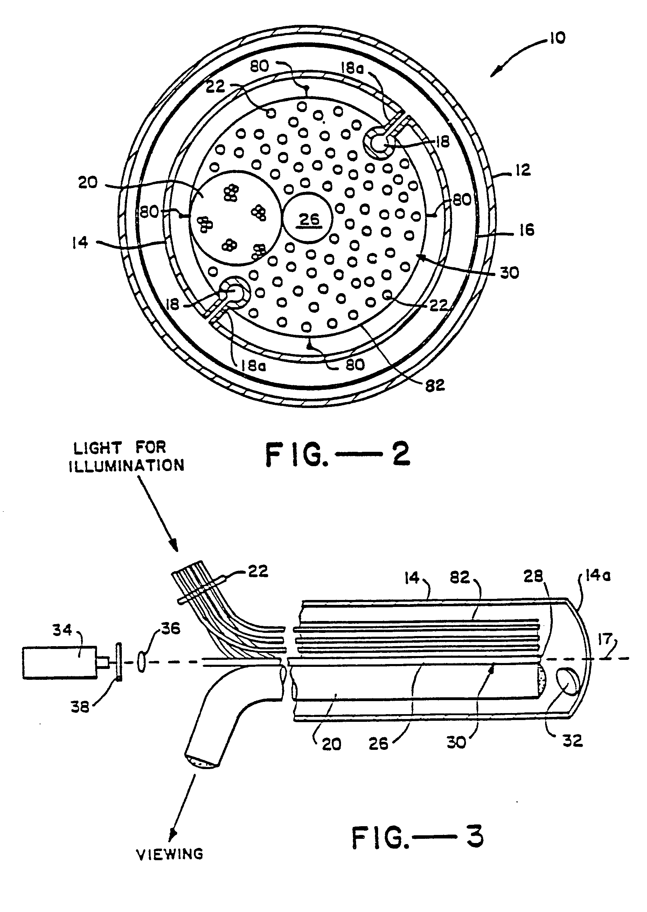

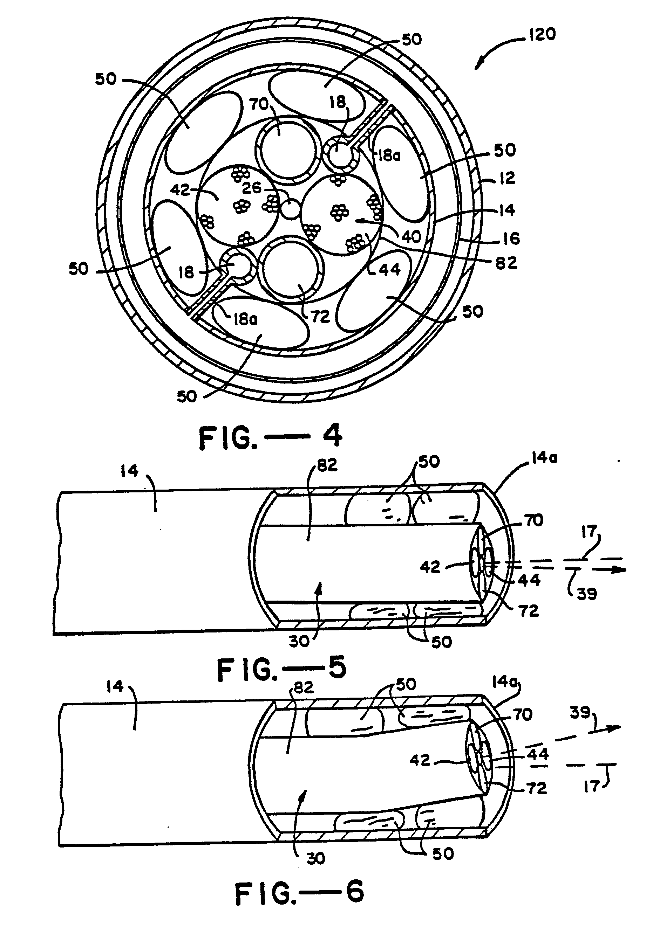

To illuminate stenotic obstruction 92, an optical fiber bundle comprising a plurality of optical fibers 22 is provided within catheter 14, see Figures 2 and 3. Optical fibers 22 originate at an exterior intense viewing light source, which is not illustrated, and extend from the proximal end 14b of catheter 14 to a point within the catheter near its distal end 14a. Optical fibers 22 are used to illuminate the area in front of the distal end of catheter 14.

Catheter 14 further includes an optical fiber bundle 20 consisting of a plurality of optical fibers for viewing the area in front of distal end 14a. Bundle 20 is connected to an appropriate eyepiece, which is not illustrated, and extends from the proximal end 14b of catheter 14 to terminate at a point within the catheter near distal end 14a. If desired, a protective transparent shield may be provided over the distal end of bundle 20. Viewing bundle 20 forms an image that is produced and viewed by conventional means as in a medical endoscope. Bundle 20, which is offset from central axis 17 of catheter 14, collects light through a tilted lens 32. Lens 32 permits observation of the central portion of the artery, the surrounding area, and the occlusion.

A laser-beam transmitting fiber 26 for transmission of laser energy from a laser source 34 is also carried by catheter 14. Laser-transmitting fiber 26 extends from proximal end 14b of catheter 14 to a point inwardly of but near the distal end of catheter 14. Fiber 26 is preferably located along the central axis of catheter 14, and may also be covered by a transparent shield if desired.

Laser 34 is coupled to laser-transmitting fiber 26 by a lens 36, which is either placed between the laser and the laser-transmitting fiber, as illustrated, or which is incorporated into the optical fiber. The exterior surface of laser transmitting fiber 26 is coated with an anti-reflective coating for the laser wavelength of interest. A shutter 38 between laser 34 and lens 36 permits control of laser irradiation. Endoscopes used in conjunction with lasers for the performance of surgery are discussed in U.S. Patent 3,858,577, issued January 7, 1975, Bass, et al., and U.S. Patent 4,146,019, issued March 27, 1979, Bass, et al. The disclosure in these two patents are hereby incorporated by reference.

Laser 34 has to deliver to the target area sufficient power at the predetermined wavelength to destroy, vaporize or soften plaque material 92. The laser beam should be conducted through optical fiber 26 with as little power loss as feasible. Additionally, the laser should interact with the plaque material rather than with the surrounding normal tissue of the artery or any infrared liquid that may be present. Several types of lasers can be used, for example, argon-ion, carbon dioxide, and Neodynium YAG lasers. Lasers having a wavelength capable of destroying or softening plaque but without adverse effect on blood cells or tissue are preferred. For lasers of certain wavelengths, for instance, carbon dioxide lasers, some laser-transmitting guide other than an optical fiber might have to be used.

To achieve the high intensities required for destruction of plaque material 92, the laser is focused by a short-focal length lens 28. Lens 28 is either spaced from the end of optical fiber 26, or, as shown, affixed to the end of optical fiber 26. Lens 28 may comprise a plurality of lenses.

The laser will destroy blood cells present and may produce blood clotting in the area of the targeted occlusion. Clotting in the occluded coronary artery would further compromise blood flow to the heart. Thus, laser surgery in the coronary artery requires that blood flow to the targeted occlusion be stopped. Catheter assembly 10 includes an inflatable or expandable balloon 16 for temporarily stopping the flow of blood into the area in front of the distal end of catheter 14. Blood flow in the coronary artery, however, cannot be interrupted for more than 12 to 15 seconds without endangering the patient's life. By temporarily interrupting the flow of blood, blood can be excluded from the region, the obstructed or constricted area can be viewed, and the obstruction can be successfully removed. The region in front of the distal end of catheter 14 can also be flushed with a solution, such as Ringer's or saline, to dilute any blood present in that region. Perhaps, a gas such as carbon dioxide could also be injected into this region to expand the artery, and to clear blood from the ends of the optical fibers.

Inflatable balloon 16 is circumferentially affixed about the exterior surface of catheter 14, and is located near the distal end of catheter 14. Prior to opening shutter 38, balloon 16 is inflated to sealingly engage the interior walls of coronary artery 90, thereby stopping the flow of blood into the area of targeted occlusion 92, see Figure 1C. The laser will vaporize the occlusion within a time period of less than one second depending upon the type and power of laser used. Afterwards, balloon 16 may be deflated to permit blood flow through the coronary artery, or if time permi'ts, catheter 14 may be extended further to remove or soften an additional section of plaque before the flow of blood is restored.

As balloon 16 only has to stop the flow of blood and not expand the artery, the balloon can be inflated by lower pressures and be constructed of less rigid materials than a "Grüntzig"-type balloon. Alternatively, balloon 16 may be constructed to stop blood flow as well as to expand soft plaque or any other deformable construction in an artery.

In an alternate embodiment, as illustrated in Figure 8, catheter assembly 10 may have inflatable means 16 circumferentially affixed about the exterior surface of catheter 12 near the distal end 12a thereof. This embodiment is particularly useful in the removal of plaque or clot 102 found in a peripheral vessel 100, such as the iliar and femoral arteries.

A pair of channels 18, see Figure 2, extend through catheter 14 and are connected to the interior of balloon 16 by means of a flexible tubing 18a. A gas, such as carbon dioxide, or a liquid, such as saline, is passed through channels 18 from a source, which is not illustrated, to expand balloon 16. To deflate the balloon, the gas or fluid may be withdrawn from the balloon by means of these channels. Channels 18 may also be built into the wall of catheter 14.

A cable pulley arrangement is provided to position catheter 14 which carries the laser, illumination, and viewing fiber optics. As shown in Figure 2, the cables are attached at appropriate points 80 to a sheath 82 which is disposed circumferentially about optical fibers 22, viewing bundle 20, and laser fiber 26. Sheath 82 may extend the length of the optical fibers back to proximal end 14b of catheter 14. Sheath 82 defines a grouping 30 of fiber optics within the central section of catheter 14. The grouping includes viewing bundle 20, laser optical fiber 26, and illumination optical fibers 22. As in gastroendoscopy, the cable pulley arrangement can be operated to tilt grouping 30 or to cause it to move transversely relative to the central axis of the catheter assembly. This permits viewing and laser irradiation of sites in the artery which are not centrally located with respect to catheter 14. Alternatively, as shown in Figure 8, cables 81 of the cable pulley arrangement may be attached at appropriate points 80 to the outside surface of catheter 14 at the distal end thereof. The illumination fiber optics of catheter 10 have been described as comprising a plurality of individual optical fibers 22 disposed within catheter 14. It is to be understood, however, that a fiber optical bundle, like bundle 20, could be fashioned to include both illumination and viewing optical fibers. Such an approach is described below.

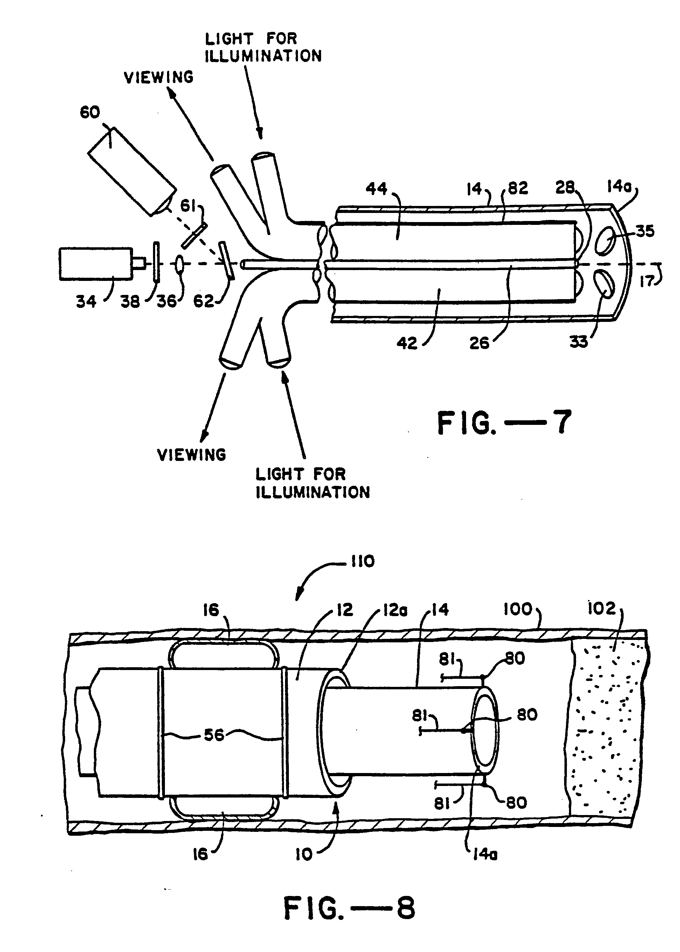

An alternative embodiment of the catheter assembly of the present invention is illustrated in Figures 4 through 7. In that embodiment, catheter assembly 120 includes two fiber optical bundles 42 and 44 to provide binocular vision, and thus depth perception of the target area. Each bundle 42 and 44 in turn includes an illumination bundle portion connected to an intense light source for illuminating the area in front of distal end 14a, and a viewing bundle portion connected to an eyepiece for viewing the area in front of distal end 14a.

Bundles 42 and 44 are located on either side of the central axis of catheter 14, and they collect light through their own respective tilted lenses 33 and 35. The lenses are suitably tilted to allow observation of the central portion of the artery as well as the surrounding area. This stereoscopic binocular approach permits perception of depth within the artery. As in the embodiment described above, bundles 42 and 44 permit observation of a wide area of the coronary artery, as well as the obstruction and laser focal spot.

The embodiment illustrated in Figure 7 also includes an intense coherent light source 60 which is located between laser 34 and laser fiber optic 26. Light source 60 is slightly offset from the laser propagation axis. Light source 60 permits viewing of the precise region of the laser focus as well as the exact size of the focused laser beam. An optical filter 61 and a beam splitter 62 are located between light source 60 and laser fiber optic 26 to help distinguish the laser focal region from the region of general illumination. Light source 60 serves as an aiming beam or target light when lasers having an invisible wavelength are used.

Catheter assembly 120 further includes a flushing tube 70 and a suction tube 72. Both of these tubes extend within catheter 14 from its proximal end 14b to a point inwardly of distal end 14a, see Figures 4 through 6.

Flushing tube 70 is provided for injecting a flushing fluid, such as saline or Ringer's solution, into the area between the distal end of catheter 14 and the occlusion. The flushing fluid can be injected to dilute and/or remove any trapped blood, after blood flow to the occlusion is stopped by balloon 16. A radio-opague dye may also be injected into this area through flushing tube 70 so that the dye may be viewed on a fluoroscope to determine the site of the obstruction. A gas such as carbon dioxide could also be injected through flushing tube 70. Suction tube 72, which is connected to an appropriate filtering system outside of catheter 14, is desinged to remove the aforesaid diluted blood and flushing fluid, dye, or products that may form after the plaque material has been vapored or otherwise removed by the laser.

Grouping 30 of catheter assembly 120 includes laser-transmitting fiber optic 26, and fiber optic bundles 42 and 44. Grouping 30 also includes flushing and suction tubes 70 and 72. To position grouping 30, a series of positioning balloons 50 are disposed circumferentially about the grouping. Inflatable balloons 50 are each operably connected to independent pressure sources, which are not illustrated, via tubes, also not illustrated, extending from the proximal end of catheter 14. As with the cable pulley arrangement, inflatable balloons 50 permit grouping 30 to move transversely and tilt relative to the central axis of the catheter assembly. The positioning-balloon system permits viewing and laser irradiation of sites that are not centrally located relative to the central axis of catheter 14.

As shown in Figures 5 and 6, where reference numeral 39 represents the central axis of grouping 30, by inflating some balloons and deflating others, grouping 30 can be appropriately positioned relative to the target area. Figure 5 shows an arrangement where grouping 30 has been moved transversely with respect to the central axis of the catheter assembly. Figure 6 shows an arrangement in which grouping 30 has been moved transversely and tilted.

In other embodiments, the catheter assembly of the present invention could employ more than one laser.

The laser-transmitting fiber could also be offset from the central axis of the inner catheter and could use a tilted or nonsymmetrical lens. Different configurations of the viewing and illuminating optical fibers are also possible.

Although certain specific embodiments of the invention have been described herein in detail, the invention is not to be limited to only such embodiments, but rather only by the appendant claims.

**Claims**

1. A catheter assembly, comprising an outer catheter (12) for insertion into a body channel, said outer catheter having distal and proximal ends (12a; 12b), an inner catheter (14) positionable within said outer catheter and having a distal end (14a) that is extendable toward the distal end (12a) of said outer catheter, an optical fiber (26) extending through a portion or said inner catheter (14) for transmitting laser energy therethrough for removing an obstruction outwardly of the distal end of said inner catheter, and optical fibers (20, 22) extending through a portion of said inner catheter (14) for illuminating and viewing the area in front of the distal end (14a) of said inner catheter; characterised in that an inflatable means (16) associated with one of said catheters is provided for effecting a seal with the interior wall of the body channel to prevent the flow of a body fluid therepast so as to facilitate laser irradiation of the obstruction outwardly of the distal end (14a) of said inner catheter (14).

2. A catheter assembly as claimed in Claim 1, characterised in that means (50, 80, 82; 80, 81) are provided for positioning said optical fibers (20, 22) relative to the axis of the outer catheter.

3. A catheter assembly of Claim 2, characterised in that said positioning means includes a cable (81) and pulley arrangement.

4. A catheter assembly as claimed in any of Claims 1 to 3, characterised in that said optical fibers for illuminating and viewing include a plurality of optical fibers (22) extending through a portion of said inner catheter (14) and terminating within said inner catheter near the distal end (14a) thereof for use in illuminating the area in front of the distal end of said inner catheter; and a fiber optic bundle (20) extending through a portion of said inner catheter (14) and terminating within said inner catheter near the distal end thereof for use in viewing the area in front of the distal end of said inner catheter.

5. A catheter assembly as claimed in any of Claims 1 to 4, characterised in that said optical fibers comprise a fiber optical bundle having a viewing bundle portion (20) for viewing the area in front of the distal end (14a) of said inner catheter (14), and a light source bundle portion (22) for illuminating said area.

6. A catheter assembly as claimed in Claim 5, characterised in that there are two of said fiber optical bundles (42, 44) disposed on opposite sides of the central axis of said inner catheter (14).

7. A catheter assembly as claimed in any of Claims 1 to 5, characterised in that said laser fiber (26) is positioned along the central axis of said inner catheter (14).

8. A catheter assembly as claimed in any of the preceding claims and further including a flushing channel (70) extending through a portion of said inner catheter (14) and terminating within said inner catheter near the distal end (14a) thereof.

9. A catheter assembly as claimed in any preceding claims and further including a suction channel (72) extending through a portion of said inner catheter (14) and terminating within said inner catheter near the distal end (14a) thereof.

10. A catheter assembly as claimed in any of Claims 1 to 9, characterised in that the end of said laser fiber (26) terminating near the distal end (14a) of said inner catheter (14) includes a lens (28) for focusing the laser beam on the obstruction.

11. A catheter assembly as claimed in any of Claims 1 to 10, and further including means (50; 80; 81; 82) for positioning said laser optical fiber (26) relative to the central axis of said outer catheter (14).

12. A catheter assembly as claimed in Claim 11, characterised in that said positioning means for said laser optical fiber includes a cable and pulley arrangement (80; 81).

**Patentansprüche**

1. Katheterbaugruppe, bestehend aus einem Außenkatheter (12) zum Einfügen in einen Mittelkanal, wobei dieser Außenkatheter mit je einem distalen und proximalen Ende (12a; 12b) versehen ist, einem innerhalb des erwähnten Außenkatheters verschiebbaren und mit einem in Richtung auf das distale Ende (12a) des erwähnten Außenkatheters ausziehbaren distalen Ende (14a) versehener Innenkatheter (14), eine optische Glasfaser (26), die durch einen Abschnitt des erwähnten Innenkatheters (14) verläuft, um zur Beseitigung einer Verstopfung außerhalb des distalen Endes des erwähnten Innenkatheters Laserenergie hierdurch zu leiten, und aus optischen Glasfasern (20, 22), die durch einen Abschnitt des erwähnten Innenkatheters (14) verlaufen, um den Bereich vor dem distalen Ende (14a) des erwähnten Innenkatheters zu beleuchten und in Augenschein zu nehmen, dadurch gekennzeichnet, daß eine an einen der Katheter angeschlossene aufblasbare Vorrichtung (16) vorgesehen ist, um eine Abdichtung gegen die Innenwand des Mittelkanals zu bewirken, damit verhindert wird, daß Körperflüssigkeit daran vorbeifließen kann, und um so die Laserbestrahlung der Verstopfung außerhalb des distalen Endes (14a) des erwähnten Innenkatheters (14) zu ermöglichen.

2. Katheterbaugruppe nach Anspruch 1, dadurch gekennzeichnet, daß Vorrichtungen (50, 80, 82; 80, 81) vorgesehen sind, um die erwähnten optischen Glasfasern (20, 22) in Bezug auf die Achse des Außenkatheters zu verschieben.

3. Katheterbaugruppe nach Anspruch 2, dadurch gekennzeichnet, daß zu der erwähnten Verschiebevorrichtung ein Kabel (81) und eine Laufrollenvorrichtung gehören.

4. Katheterbaugruppe nach einem der Ansprüche 1 bis 3, dadurch gekennzeichnet, daß zu den erwähnten optischen Glasfasern zur Beleuchtung und zur Inaugenscheinnahme eine Vielzahl von optischen Glasfasern (22), die durch einen Abschnitt des erwähnten Innenkatheters (14) verlaufen, innerhalb des erwähnten Innenkatheters in der Nähe des distalen Endes (14a) hiervon enden und zur Beleuchtung des Bereichs vor dem distalen Ende des erwähnten Innenkatheters dienen, sowie ein optisches Glasfaserbündel (20) gehören, das durch einen Abschnitt des erwähnten Innenkatheters (14) verläuft, innerhalb des erwähnten Innenkatheters in der Nähe des distalen Endes hiervon endet und zur Inaugenscheinnahme des Bereichs vor dem distalen Ende des erwähnten Innenkatheters dient.

5. Katheterbaugruppe nach einem der Ansprüche 1 bis 4, dadurch gekennzeichnet, daß die erwähnten optischen Glasfasern ein optisches Glasfaserbündel mit einem Sichtbündelabschnitt (20) zur Inaugenscheinnahme des Bereichs vor dem distalen Ende (14a) des erwähnten Innenkatheters (14) und mit einem Lichtquellenbündelabschnitt (22) zur Beleuchtung des erwähnten Bereichs umfaßt.

6. Katheterbaugruppe nach Anspruch 5, dadurch gekennzeichnet, daß zwei von den erwähnten optischen Glasfaserbündel (42, 44) vorhanden sind, die jeweils gegenüberliegend auf der Mittelachse des erwähnten Innenkatheters (14) angeordnet sind.

7. Katheterbaugruppe nach einem der Ansprüche 1 bis 5, dadurch gekennzeichnet, daß die erwähnte Laserfaser (26) entlang der Mittelachse des erwähnten Innenkatheters (14) verschoben wird.

8. Katheterbaugruppe nach einem der vorstehenden Ansprüche, zu dem ferner ein Spülkanal (70) gehört, der durch einen Abschnitt des erwähnten Innenkatheters (14) verläuft und innerhalb des erwähnten Innenkatheters in der Nähe des distalen Endes (14a) hiervon endet.

9. Katheterbaugruppe nach einem der vorstehenden Ansprüche, zu dem ferner ein Saugkanal (72) gehört, der durch einen Abschnitt des erwähnten Innenkatheters (14) verläuft und innerhalb des erwähnten Innenkatheters in der Nähe des distalen Endes (14a) hiervon endet.

10. Katheterbaugruppe nach einem der Ansprüche 1 bis 9, dadurch gekennzeichnet, daß zu dem Ende der erwähnten Laserfaser (26), welche in der Nähe des distalen Endes (14a) des erwähnten Innenkatheters (14) endet, eine Linse (28) gehört, um den Laserstrahl auf die Verstopfung zu bündeln.

11. Katheterbaugruppe nach einem der Ansprüche 1 bis 10, zu dem ferner Vorrichtungen (50; 80; 81; 82) zum Verschieben der erwähnten optischen Laserfaser (26) in Bezug auf die Mittelachse des erwähnten Außenkatheters (14) gehören.

12. Katheterbaugruppe nach Anspruch 11, dadurch gekennzeichnet, daß zu der erwähnten Verschiebevorrichtung für die erwähnte optische Laserfaser eine Kabel- und Laufrollenvorrichtung (80; 81) gehört.

## Revendications

1. Ensemble cathéter comprenant un cathéter extérieur (12) pour introduction dans un canal du corps, ledit cathéter extérieur ayant des extrémités distale et proximale (12a; 12b), un cathéter intérieur (14) pouvant être placé à l'intérieur dudit cathéter extérieur et ayant une extrémité distale (14a) que l'on peut faire s'étendre vers l'extrémité distale (12a) dudit cathéter extérieur, une fibre optique (26) s'étendant par une partie dudit cathéter intérieur (14) pour la transmission d'énergie laser par lui en vue de l'élimination d'une obstruction au delà de l'extrémité distale dudit cathéter intérieur, et des fibres optiques (20, 22) s'étendant par une partie dudit cathéter intérieur (14) pour éclairer et examiner la région en avant de l'extrémité distale (14a) dudit cathéter intérieur, caractérisé en ce qu'un moyen dilatable (16) associé à un desdits cathéters est prévu pour assurer l'étanchéité avec la paroi intérieure du canal du corps pour empêcher l'écoulement d'une humeur ou sécrétion le long de lui de manière à faciliter l'irradiation par laser de l'obstacle au delà de l'extrémité distale (14a) dudit cathéter intérieur (14).

2. Ensemble cathéter selon la revendication 1, caractérisé en ce que des moyens (50, 80, 82; 80, 81) sont prévus pour la mise en place desdites fibres optiques (10, 22) par rapport à l'axe du cathéter extérieur.

3. Ensemble cathéter selon la revendication 2, caractérisé en ce que lesdits moyens de mise en place comprennent un dispositif câble (81) et poulie.

4. Ensemble cathéter selon l'une quelconque des revendications 1 à 3, caractérisé en ce que lesdites fibres optiques d'éclairage et d'examen comprennent une pluralité de fibres optiques (22) s'étendant par une partie dudit cathéter intérieur (14) et se terminant à l'intérieur dudit cathéter intérieur près de son extrémité distale (14a) pour emploi comme éclairage de la région devant l'extrémité distale dudit cathéter intérieur; et un faisceau de fibres optiques (20) s'étendant par une partie dudit cathéter intérieur (14) et se terminant à l'intérieur dudit cathéter intérieur près de son extrémité distale pour emploi à l'examen de la région en avant de l'extrémité distale dudit cathéter intérieur.

5. Ensemble cathéter selon l'une quelconque des revendications 1 à 4, caractérisé en ce que lesdites fibres optiques comprennent un faisceau de fibres optiques ayant une partie de faisceau d'inspection (20) pour l'examen de la région en avant de l'extrémité distale (14a) dudit cathéter intérieur (14), et une partie de faisceau source de lumière (22) pour éclairer ladite région.

6. Ensemble cathéter selon la revendication 5, caractérisé en ce qu'il est prévu deux desdits faiceaux de fibres optiques (42, 44) placés sur des côtés opposés dudit axe central dudit cathéter intérieur (14).

7. Ensemble cathéter selon l'une quelconque des revendications 1 à 5, caractérisé en ce que ladite fibre laser (26) est placée le long de l'axe central dudit cathéter intérieur (14).

8. Ensemble cathéter selon l'une quelconque des revendications précédentes et comprenant en outre un canal (70) de nettoyage par écoulement de fluide s'étendant par une partie dudit cathéter intérieur (14) et se terminant à l'intérieur dudit cathéter intérieur près de son extrémité distale (14a).

9. Ensemble cathéter selon l'une quelconque des revendications précédentes et comprenant en outre un canal d'aspiration (72) s'étendant par une partie dudit cathéter intérieur (14) et se terminant à l'intérieur dudit cathéter intérieur près de l'extrémité distale (14a) de ce dernier.

10. Ensemble cathéter selon l'une quelconque des revendications 1 à 9, caractérisé en ce que l'extrémité de ladite fibre laser (26) se terminant près de l'extrémité distale (14a) dudit cathéter intérieur (14) comprend une lentille (28) pour la focalisation du faisceau laser sur l'obstacle.

11. Ensemble cathéter selon l'une quelconque des revendications 1 à 10, et comprenant en outre des moyens (50; 80; 81; 82) de mise en place de ladite fibre optique laser (26) par rapport à l'axe central dudit cathéter extérieur (14).

12. Ensemble cathéter selon la revendication 11, caractérisé en ce que ledit moyen de mise en place pour ladite fibre optique laser comprend un dispositif câble et poulie (80, 81).

EP 0 094 964 B1

FIG.—IA

FIG.—IB

FIG.—IC

1

FIG. — 2

LIGHT FOR ILLUMINATION

VIEWING

FIG. — 3

FIG. — 4

FIG. — 5

FIG. — 6

LIGHT FOR ILLUMINATION

VIEWING

60

61

34  38  36  62

44  14  82  28  14a

35

17

42  26  33

VIEWING

LIGHT FOR ILLUMINATION

FIG. — 7

110

16  12  12a  14  100  102

81  80

56  81  80

81  80

16  10  81  80

14a

FIG. — 8

4